# EUROPEAN PATENT APPLICATION

(11) **EP 2 446 851 A2**
(43) Date of publication of application: **02.05.2012**
(21) Application number: 11187054.9
(22) Date of filing: 28.10.2011
(51) Int. Cl.: A61B 19/00

(54) **One touch button for operating room support**

(30) Priority: 28.10.2010 US 407617 P
(71) Applicant: Arthrex, Inc., Naples, FL 34108-1945 (US)
(72) Inventor: Seifert, Alexander Jorg, Naples, Florida 34109 (US)
(74) Representative: Shanks, Andrew

(57) **Abstract**

A method of obtaining assistance during a surgical procedure includes the steps of requesting assistance with a single action and establishing a line of communication between an individual in an operating room and an individual at a remote location in response to the step of requesting assistance. The method includes the steps of communicating a question to the individual at the remote location and receiving an answer to the question from the individual at the remote location.

## Description

### BACKGROUND OF THE INVENTION

During surgery, a surgeon may have questions or require technical support. For example, a surgeon may require technical support relating to the use of a particular surgical instrument, such as how the instrument is to be used during a specific procedure for a specific situation, or may require technical support on the use or troubleshooting of an electronic device. In this case, the surgeon or other operating personal must contact a technician or sales representative, who specializes in the surgical instrument by phone, or wait for them to arrive at the clinic, to discuss or help resolve the question or problem. This can require the surgeon or medical personal to talk to several people before speaking to the desired person, which increases frustration, stress and operating room time. On occasion, a surgeon may need the professional consulting services of another physician who has more clinical experience in a new surgical technique.

### SUMMARY OF THE INVENTION

A method of obtaining assistance during a surgical procedure includes the steps of requesting assistance with a single action and establishing a line of communication between an individual in an operating room and an individual at a remote location in response to the step of requesting assistance. The method includes the steps of communicating a question to the individual at the remote location and receiving an answer to the question from the individual at the remote location.

An assistance system for use during a surgical procedure includes an operating room computer in an operating room associated with an operating room communication device and a remote location computer in a remote location with a remote location communication device. The assistance system also includes a button associated with the operating room computer. When activated, the button sends a signal that opens a line of communication between the operating room computer and the remote location computer. The individual in the operating room and the individual in the remote location communicate over the line of communication using the operating room communication device and the remote location communication device, respectively.

These and other features of the present invention will be best understood from the following specification and drawings.

### BRIEF DESCRIPTION OF THE DRAWINGS

Figure 1 illustrates an operating room; and
Figure 2 illustrates a computer in the operating room.

### DETAILED DESCRIPTION OF THE PREFERRED EMODIMENT

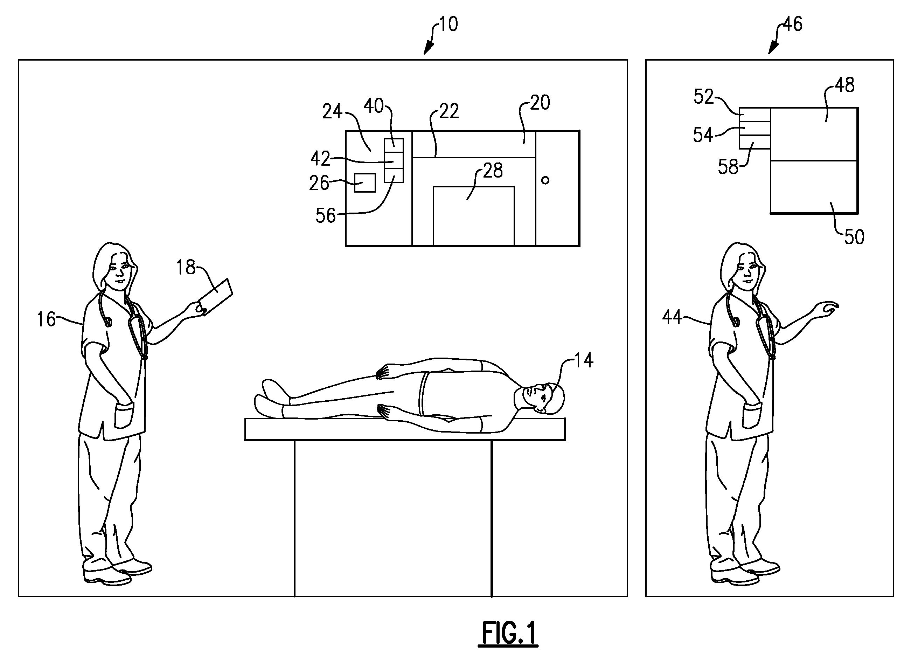

Figure 1 illustrates an operating room 10 including a surgical table 12, a patient 14 located on the surgical table 12, and a surgeon 16 or other operating room personal. A surgeon 16 can use a surgical instrument 18 during a surgical procedure. The operating room 10 also includes an all in one equipment management and visualization system 20 (shown schematically). In one example, the system 20 includes shelves 22 and cabinets 24 for medical and video equipment including booms and extension arms. In one example, the system 20 is a NUBOOM® or NUBOOM Ultra system, which is manufactured by Compview Medical. NUBOOM® is a registered trademark of Comp-View, Inc. of Beaverton, Oregon.

The system 20 includes a virtual or mechanical one-touch button 26 that can be pressed or touched during a surgical procedure to send a signal to open a line of communication with a physician or technician 44 at a remote location 46. If mechanical, the button 26 can be pressed from an original position to an activation position and then biased back to the original position when released. The button 26 can also be simply touch activated.

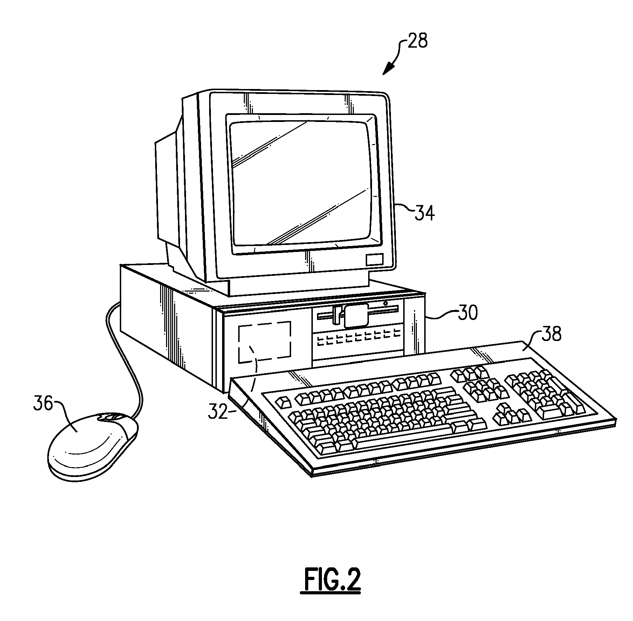

The system 20 can also include a computer 28 (shown in Figure 2) having a microprocessor or CPU 30, a storage 32 (memory, hard drive, optical, and/or magnetic, etc.), a display 34, a mouse 36, a keyboard 38 and other hardware and software for performing the functions described herein. The system 20 also includes a camera 40, a microphone 42, and a speaker 56 associated with the computer 28. The camera 40 associated with the computer 28 is capable of taking images that can be viewed by the surgeon or technician 44 on a display 48. The microphone 42 and a speaker 56 allow the surgeon 16 to speak with the technician or physician 44 to over the Internet or teleconference system.

In one example, the camera 40 is a digital camera. In one example, the camera 40 is a video camera. For example, the camera 40 is a web camera. However, any type of camera 40 can be employed. The camera 40 is capable of taking images of the operating room 10 or endoscopic images inside the patient 14. The images can be a photographic image (still image) or a video image (motion image).

When the button 26 is pressed or touched by the surgeon 16 or other medical personal, a signal is sent to a computer 50 at the remote location 46, opening a line of communication between the operating room 10 and the remote location 46 for immediate assistance. The individuals in the operating room 10 can obtain an immediate connection and full-time help from a technician or physician 44 or concierge at the remote location 46. In one example, the connection is made over the Internet. In another example, the connection is made using a secure broadband connection similar to Cisco® Systems Telepresence, allowing for higher resolution imaging and audio transmission with lower rate of transmission disruption. Cisco® is a registered trademark of Cisco Technology, Inc., of San Jose, California.

When the button 26 is pressed or touched, the surgeon 16 or medical personal can obtain several types of help. Activating or pressing the button 26 sends a signal that requests assistance during a surgical procedure. In one example, the button 26 can be pressed to provide immediate technical support. One touch of the button 26 can activate a one-touch, on-screen video teleconference line between the surgeon 16 and the technician or physician 44 at the remote location 46. In another example, immediate audio-visual support can be provided during the surgery. In a third example, the system 20 can be used to provide online marketing, technical data or surgical steps by accessing a library of materials. The support options could be developed based on a subscription fee and a menu of "service levels." Alternately, fees could be charged based on use.

During surgery, it is possible that a situation could arise where a surgeon 16 or medical personnel could not get into contact with the local sales representative when support or assistance is needed. Instead of calling multitudes of people, the button 26 could be pressed to connect the surgeon 16 immediately and directly with the technician or physician 44. The concierge physician or technician 44 can then troubleshoot the problem and discuss solutions with the surgeon 16. The problem can be discussed over a teleconference system, a video teleconference system, a telephone or over the Internet. Or, in the event the concierge physician or technician 44 cannot resolve the problem remotely, the concierge physician or technician 44 can contact a local technician on behalf of the operating room team, acting as a "concierge operator" and help locator, to ensure a local sales representative, service representative, technician or manager is aware of the problem and can assist in scheduling a time for the local human resource to provide support.

If the surgeon 16 needs technical assistance during surgery, the camera 40 can obtain images of the surgeon 16, the patient 14, the instrument 18, or any other item or person in the operating room 10 which can be viewed by the technician or physician 44 at the remote location 46. In one example, the images taken by the camera 40 are displayed on a display 48 at the remote location 46 for viewing by the technician 44. The display 48 is associated with a computer 50 at the remote location 46. The computer 50 includes a microprocessor or CPU, a storage (memory, hard drive, optical, and/or magnetic, etc.), a mouse, a keyboard and other hardware and software for performing the functions described herein (the same components of the computer 28 of Figure 2). The images can be provided to the technician or physician 44 through the Internet or through other teleconference systems.

The technician or physician 44 views the images taken by the camera 40 on the display 48 to help solve problems associated with the surgery or to answer the questions of the surgeon 16. In one example, the camera 40 includes the microphone 42 that allows the surgeon 16 to speak over the Internet or through the teleconference system.

The remote location 46 also includes a camera 52 associated with the computer 50 that is capable of taking images of the technician or physician 44 that can be viewed by the surgeon 16 on the display 34. In one example, the camera 52 is a digital camera. In one example, the camera 52 is a video camera. For example, the camera 52 is a web camera. However, any type of camera 52 can be employed. A microphone 54 and a speaker 58 associated with the computer 50 also allow the technician or physician 44 to speak to the surgeon 16 over the Internet or teleconference system.

After the button 26 is pressed, a signal is sent to open an immediate line of communication between the operating room 10 and the remote location 46 so that the technician or physician 44 can immediately respond the surgeon 16. The surgeon 16 and the technician or physician 44 can then discuss questions over the Internet or teleconference system, using the computers 28 and 50, the cameras 40 and 52, the microphones 42 and 54, and the speakers 56 and 58 to solve the problem or answer any questions. As only a single press of the button 40 is needed to obtain and open a connection, the surgeon 16 can quickly and immediately be connected to the technician or physician 44 to obtain assistance. The technician or physician 44 can also act as an operator switch coordinator, passing the surgeon 16 to another physician, another technician, or anyone else for direct assistance.

The one-touch-button 26 helps connect physicians via video-teleconference to provide "tele-consulting" between physicians or individuals who are geographically far apart, but who can benefit from technology that allows the consulting physician (in another location of the hospital, country or even continent) to interact live and provide surgical guidance to the surgeon 16.

The foregoing description is only exemplary of the principles of the invention. Many modifications and variations are possible in light of the above teachings. It is, therefore, to be understood that within the scope of the appended claims, the invention may be practiced otherwise than using the example embodiments which have been specifically described. For that reason the following claims should be studied to determine the true scope and content of this invention.

## Claims

1. A method of obtaining assistance during a surgical procedure, the method comprising the steps of:
requesting assistance during a surgical procedure in an operating room with a single action;
establishing a line of communication between an individual in the operating room and an individual at the remote location in response to the step of requesting assistance;
communicating a question to the individual at the remote location; and
receiving an answer to the question from the individual at the remote location.

2. The method of claim 1 wherein the step of requesting assistance includes pressing or touching a button.

3. The method of claim 1 or 2 wherein the step of requesting assistance activates an audio-video communication line between an individual in the operating room and the individual at the remote location.

4. The method of any preceding claim wherein the step of requesting assistance includes requesting technical support.

5. The method of any preceding claim wherein the step of communicating the question to the individual at the remote location includes sending visual images of at least one of a surgeon, a patient or a surgical instrument to the individual at the remote location to assist in answering the question.

6. The method of any preceding claim wherein the step of communicating the question to the individual at the remote location includes communicating with the individual at the remote location employing audio signal.

7. The method of any preceding claim wherein the step of receiving an answer to the question includes obtaining technical support, technical data or surgical steps from a library of materials.

8. The method of any preceding claim wherein the step of establishing a line of communication occurs immediately after the step of requesting assistance.

9. An assistance system for use during a surgical procedure comprising:
an operating room computer in an operating room, wherein the computer is associated with an operating room communication device;
a remote location computer in a remote location, wherein the remote location computer is associated with a remote location communication device; and
a button associated with the operating room computer in the operating room, wherein activating the button sends a signal that opens a line of communication between the operating room computer and the remote location computer, wherein the individual in the operating room and the individual in the remote location communicate over the line of communication using the operating room communication device and the remote location communication device, respectively.

10. The assistance system of claim 9 the button is pressed or touched to immediately open the line of communication.

11. The method of any of claims 1 to 8 or the assistance system of claim 9 or 10 wherein the individual in the operating room is a surgeon and the individual in the remote location is a technician or a physician.

12. The method of any of claims 1 to 8, or 11, or the assistance system of claim 9, 10 or 11 wherein the step of communicating is performed or the line of communication occurs overs the Internet, a secure broadband connection, or a telephone.

13. The assistance system of any of claims 9 to 12 wherein the operating room communication device comprises an operating room camera and an operating room display and the remote location communication device comprises a remote location camera and a remote location display, wherein images of the operating room and the remote location are communicated over the line of communicate such that the individual in the operating room can view images of the remote location obtained by the remote location camera, and the individual in the remote location can view images of the operating room obtained by the operating room camera, and optionally wherein the camera captures images of at least one of the individual in the operating room, the individual in the remote location, a patient, and a surgical instrument.

14. The assistance system of any of claims 9 to 13 wherein the operating room communication device comprises an operating room microphone and an operating room speaker and the remote location communication device comprises a remote location microphone and a remote location speaker, wherein audio of the individual in the operating room and the individual in the remote location are communicated over the line of communication such that the individual in the operating room can hear audio of the individual in the remote location obtained by the remote location microphone over the operating room speaker, and the individual in the remote location can hear audio of the individual in the operating room obtained by the operating room microphone over the remote location speaker.

15. The assistance system of any of claims 9 to 14 wherein the individual in the operating room communicates a question over the communication system and the individual at the remote locate communicates an answer including at least one of technical support, technical data or surgical steps over the communication system.
